# FASCICULE DE BREVET EUROPEEN

(11) **EP 0 554 344 B1**
(45) Date de publication et mention de la délivrance du brevet: **12.08.1998**
(21) Numéro de dépôt: 91919512.3
(22) Date de dépôt: 23.10.1991
(51) Int. Cl.: C07K 14/705

(54) **COMPOSITION PHARMACEUTIQUE POUR LE TRAITEMENT OU LA PREVENTION D'UNE TUMEUR MALIGNE**
ARZNEIMITTELZUSAMMENSETZUNG ZUR BEHANDLUNG ODER VORBEUGUNG MALIGNER TUMORE
PHARMACEUTICAL COMPOSITION FOR THE TREATMENT OR PREVENTION OF A MALIGNANT TUMOR

(30) Priorité: 23.10.1990 FR 9013101
(43) Date de publication de la demande: 11.08.1993
(73) Titulaire: TRANSGENE S.A., 67082 Strasbourg Cédex (FR)
(72) Inventeur: CHAMBON, Pierre, F-67113 Blaesheim (FR); KIENY, Marie-Paule, F-67000 Strasbourg (FR); LATHE, Richard, Leeds LS8 (GB); HAREUVENI, Mara, 47 279 Ramat-Ha-Sharon (IL)
(74) Mandataire: Warcoin, Jacques
(86) Numéro de dépôt international: FR9100835
(87) Numéro de publication internationale: WO9207000

(56) Documents cités:
- EP-A- 174 534
- EP-A- 369 816
- WO-A-88/05054
- WO-A-89/03429
- WO-A-90/05142

## Description

La présente invention a pour objet une composition pharmaceutique destinée au traitement curatif ou à la prévention d'une tumeur maligne, plus particulièrement d'un carcinome, tout spécialement d'un cancer du sein.

La plupart des cellules tumorales expriment à leur surface des antigènes qui diffèrent soit qualitativement, soit quantitativement des antigènes présents à la surface des cellules normales correspondantes. Ces antigènes sont spécifiques lorsqu'ils sont uniquement exprimés par des cellules tumorales. Lorsqu'ils sont présents à la fois sur des cellules normales et tumorales, ces antigènes sont dits associés à la tumeur; dans ce cas, ils sont présents soit en plus grande quantité, soit sous une forme différente dans les cellules tumorales.

La grande majorité des antigènes de tumeur qui ont été jusqu'à présent caractérisés chez l'homme sont des antigènes humains associés à une tumeur (appelés par la suite antigènes associés). Parmi ceux-ci on distingue:
- les antigènes oncofoetaux, tel que l'antigène carcino-embryonnaire, qui sont présents dans les tissus foetaux et absents ou à l'état de trace dans les tissus adultes correspondants; leur expression est à nouveau induite de manière aberrante lors du développement d'une tumeur;
- les antigènes de différenciation qui ne sont normalement exprimés que pendant certaines étapes de la maturation d'un type particulier de cellules; les cellules tumorales qui expriment un tel antigène auraient pour origine une cellule bloquée dans sa différenciation;
- les produits des oncogènes qui commencent à être identifiés.

La spécificité d'un antigène associé à une tumeur est donc plutôt quantitative que qualitative puisque ce dernier peut être présent chez un individu normal, de manière localisée cu intermittente (période foeto-embryonnaire) ou à l'état de traces, et ne devient hyperexprimé (expression augmentée d'un facteur 10 à 1000 fois) que lors d'un processus de tumorigenèse. Lorsque cet antigène est normalement exprimé, il est reconnu par le système immunitaire comme partie du "Soi" tandis que son hyperexpression ou son expression aberrante peut déclencher une réponse immunitaire humorale ou cellulaire.

D'une manière générale, il existe deux grands types de réponse immunitaire : la réponse de type humoral qui est caractérisée par la production d'anticorps par les lymphocytes B et la réponse immunitaire à médiation cellulaire qui met en jeu des cellules effectrices i.e., essentiellement les macrophages et les lymphocytes T cytotoxiques ainsi que des cellules régulatrices de la réponse immunitaire, i.e., les lymphocytes T helper et suppresseurs.

Une réponse immunitaire à médiation cellulaire nécessite la coopération des lymphocytes T helper et des cellules effectrices. Cette coopération s'effectue, en particulier, grâce à l'interleukine-2 et autres diverses lymphokines qui sont sécrétées par les lymphocytes T helper activés. Par la suite, l'interleukine-2 induit l'action des lymphocytes T cytotoxiques et les lymphokines déclenchent la réponse de phagocytose des macrophages. En parallèle, il existe de même un mécanisme suppresseur de la réponse immunitaire à médiation cellulaire qui met en oeuvre les lymphocytes T suppresseurs.

Il est maintenant bien connu que des patients atteints d'un cancer peuvent développer une réponse immunitaire humorale et à médiation cellulaire. Ceci a été mis en particulier en évidence en démontrant que le sérum de certains patients contenaient des anticorps anti-antigène de tumeur et que leur sérum était capable d'inhiber la croissance de cellules cancéreuses in vitro. Néanmoins, dans la mesure où les régressions tumorales spontanées sont extrêmement rares, il semble que la réponse immunitaire que l'on observe in vitro reste inefficace in vivo. Dans le même ordre d'idées, il est aussi connu que les greffes de tumeurs ne sont pas souvent rejetées, même chez des animaux immuns, tandis que les allogreffes le sont toujours.

Bien qu'une réponse immunitaire puisse se développer à l'encontre d'une tumeur, il est douteux que celle-ci soit d'un réel bénéfice pour le malade. Tout semble indiquer qu'une tumeur échappe aux mécanismes de surveillance immunitaire de l'organisme. Divers modèles ont été proposés pour expliquer ce phénomène; pour une revue complète et détaillée, voir Scientific American, Medecine, Chapter 6, VIII Tumor Immunology, 1990. En principe, les antigènes de tumeur joueraient un rôle non négligable en modifiant ou détoumant la réponse immunitaire en faveur de la tumeur plutôt qu'en faveur de l'individu.

Compte tenu de la complexité de la réponse immunitaire à l'encontre des tumeurs et de la médiocrité des connaissances actuelles dans ce domaine, la mise en oeuvre d'un vaccin anti-cancer n'est pas du tout évidente. Des études chez des animaux ont montré que l'immunisation à l'aide de cellules cancéreuses vivantes ou tuées pouvaient conduire à un rejet d'une greffe tumorale ultérieure. Des tentatives d'immunisation à l'aide de produits acellulaires ont généralement été moins réussies.

A ce jour, la possibilité de fabriquer un vaccin contre un cancer en employant un antigène associé à ce cancer reste donc controversée. Une objection théorique majeure à ce mode de traitement réside en ce qu'une réponse immunitaire ne serait pas suffisante pour prévenir ou soigner une tumeur et qu'il est fort douteux qu'un vaccin puisse être protecteur, c'est-à-dire capable d'empêcher ou de freiner le développement d'une tumeur.

Néanmoins, il a maintenant été trouvé qu'un antigène de tumeur associé, entre autre au cancer du sein peut, sous forme vaccinale ou thérapeutique, induire une réponse immunitaire qui protège contre une attaque tumorale ultérieure ou en cours de développement. Il s'agit plus précisément de l'antigène reconnu par l'anticorps monoclonal H23 issu de l'hybridome ATCC N° HB 8630, déposé aux fins de la demande de brevet EPA 174 534 et publiquement disponible pour des travaux de recherche expérimentaux. L'anticorps H23 est d'autre part commercialement disponible auprès de Teva Pharmaceutical Industries Ltd, 5 Basel Street, Petah Tiqva, P.O. Box 1424, Tel-Aviv, Israël.

L'anticorps H23 a été généré à l'encontre de matériel particulaire présent dans le surnageant des cultures in vitro de la lignée de cellules mammaires tumorales T47D. Par la suite, il a été montré que l'anticorps H23 réagissait nettement avec un grande majorité de biopsies de tumeurs mammaires ainsi qu'avec le sérum et autres liquides physiologiques des patients présentant un cancer du sein. Par contre, l'anticorps H23 ne détecte pas d'antigène, ou sinon à l'état de trace, dans le cas d'individus sains.

L'antigène de tumeur reconnu par l'anticorps H23 est donc exprimé de manière aberrante par les cellules épithéliales du tissu mammaire cancéreux dans environs 90 % des cas de cancer du sein, tandis que chez un individu normal, son expression est très faible sinon nulle. Sa présence en quantité significative a été aussi détectée dans des tissus epithéliaux tumoraux autres que les tissus épithéliaux mammaires.

Des polypeptides susceptibles d'être reconnus par l'anticorps H23 ont déjà été décrits dans l'art antérieur.

En effet, WO-A-8805054 décrit un polypeptide reconnu par un anticorps dirigé contre la partie centrale d'une mucine synthétisée par les cellules épithéliales mammaires humaines, EP-A-0 369 816 décrit un peptide reconnu par des anticorps dirigés contre une mucine humaine et WO-A-9005142 décrit des polypeptides comprenant la séquence Pro-Asp-Thr-Arg-Pro, et pouvant être inoculés directement à un homme ou un animal pour une immunisation contre la partie centrale d'une mucine.

Cependant, toutes les séquences précédemment décrites diffèrent de celle du polypeptide mis en oeuvre dans la présente invention.

En fait, chez un même patient, l'antigène de tumeur reconnu par l'anticorps H23 existe sous deux formes : une forme transmembranaire et une forme sécrétée dont les séquences en acides aminés sont respectivement montrées dans les identificateurs de séquence (IS) n° 1 et 2. La forme transmembranaire et la forme sécrétée présentent toutes deux un haut degré de polymorphisme. En effet, la séquence des deux formes d'antigène comprend une sous-unité particulière de 20 acides aminés qui apparaît encadrée dans chaque IS et qui peut être répétée en tandem plusieurs fois. La séquence de cette sous-unité a pour formule (1) : Pro-Gly-Ser-Thr-Ala-Pro-X-Ala-His-Gly-Val-Thr-Ser-Ala-Pro-Asp-Y-Arg-Pro-X dans laquelle X est Pro ou Ala et Y est Thr ou Asn. D'un individu à l'autre, le nombre de répétitions en tandem peut varier de 25 à 80 environ et, entre autre, caractérise le type polymorphe. Enfin, il peut se faire que d'une répétition à l'autre, un minimum d'acides aminés (le plus souvent 1, 2 ou 3 acides aminés) soit modifié.

D'autre part, il a été établi que la sous-unité de 2O acides aminés précédemment décrite était spécifique de l'antigène de tumeur réagissant avec l'anticorps H23 puisque cette sous-unité comporte l'épitope reconnu par cet anticorps.

En conséquence, l'invention propose une composition pharmaceutique destinée au traitement curatif ou à la prévention d'une tumeur maligne qui comprend, à titre d'agent thérapeutique, un polypeptide reconnu par l'anticorps H23 en association avec un diluent ou un support acceptable d'un point de vue pharmaceutique et dans laquelle le polypeptide reconnu par l'anticorps H23 comprend une séquence répétée n fois et de formule (I) : Pro-Gly-Ser-Thr-Ala-Pro-X₁-Ala-His-Gly-Val-Thr-Ser-Ala-Pro-Asp-Y-Arg-Pro-X₂, dans laquelle X₁ et X₂ sont indépendamment Pro ou Ala et Y est Thr ou Asn, à l'exclusion de la séquence dans laquelle X₁, X₂ et Y représentent respectivement Pro, Ala et Thr, ledit polypeptide ayant une séquence complète présentant un degré d'homologie d'au moins 80 % avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante un nombre de 1 à 80, de préférence, n étant 2, 3 ou 4.

Un polypeptide préféré reconnu par l'anticorps H23 a une séquence complète présentant un degré d'homologie d'au moins 90 %, de manière tout à fait préférée de 95 % à 100 % inclus avec (i) la séquence telle que montrée dans l'IS n° 1 ou (ii) la séquence telle que montrée dans l'IS n° 2, ces séquences étant définies ci-dessus.

De même, l'invention a aussi pour objet :
- l'usage (i) d'un polypeptide reconnu par l'anticorps H23, ou, de manière alternative, l'usage (ii) d'un virus dans le génome duquel est inséré un fragment d'ADN codant pour un polypeptide reconnu par l'anticorps H23 pour soigner ou prévenir une tumeur maligne ;
- une méthode de traitement curatif ou de prévention d'une tumeur maligne qui comprend l'acte d'administrer une quantité thérapeutiquement efficace (i) d'un polypeptide reconnu par l'anticorps H23 ou, de manière alternative, (ii) d'un virus dans le génome duquel est inséré un fragment d'ADN codant pour un polypeptide reconnu par l'anticorps H23, à un sujet ayant besoin d'un tel traitement. (Par "quantité thérapeutiquement efficace", on entend une quantité suffisante pour mettre en oeuvre une thérapie efficace).

Un polypeptide préféré reconnu par l'anticorps H23 est un polypeptide reconnu par l'anticorps H23 dont la séquence est telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; n étant un nombre de 1 à 80, de préférence n étant 2, 3 ou 4.

Plus précisément, les formes transmembranaire et sécrétée de H23 - ETA ont en commun une région N-terminale de 106 acides aminés (appelée par la suite région N-terminale) et une région médiane correspondant à l'ensemble des sous-unités répétées ; par contre leurs extrémités C-terminales divergent sensiblement. Les acides aminés de la position 107 + (20 x n) à la position 149 + (20 x n) sont identiques pour les deux formes et varient à partir de la position 150 + (20 x n).

Un polypeptide préféré reconnu par l'anticorps H23 dont la séquence n'est pas identique à l'une de celles décrites dans les IS n° 1 et 2, se caractérise par au moins une mutation d'un acide aminé (mutation ponctuelle) distribuée au hasard dans les régions N ou C-terminale. Le nombre de mutations totales doit bien sûr satisfaire le critère du degré d'homologie tel que précédemment établi. Par "mutation ponctuelle", on entend la délétion ou la substitution d'un acide aminé de la région N- ou C-terminale décrite dans l'IS n° 1 ou 2 ainsi que l'addition d'un acide aminé au sein de la région N- ou C-terminale décrite dans l'IS n° 1 ou 2.

D'une manière générale, un polypeptide reconnu par l'anticorps H23 peut étre produit par les méthodes conventionnelles de synthèse chimique ou bien, lorsque la séquence d'acides aminés comprend un nombre de résidus important, par les techniques de l'ADN recombinant. Plus particulièrement, un procédé de préparation comprend l'acte de cultiver un micro-organisme hôte transformé par un fragment d'ADN codant pour un polypeptide reconnu par l'anticorps H23 et l'acte de récolter ledit polypeptide à partir de la culture. L'organisme hôte peut être n'importe quel micro-organisme capable d'être transformé, par exemple et sans limitation, une bactérie, une levure ou bien une cellule de mammifère, dans la mesure où le fragment d'ADN considéré est soit intégré dans le génome de l'organisme hôte, soit inséré dans un vecteur d'expression approprié, c'est-à-dire, capable de se repliquer chez l'organisme hôte. Bien entendu, le fragment d'ADN codant pour le polypeptide reconnu par l'anticorps H23 est placé sous le contrôle de régions comportant des signaux de transcription et de traduction appropriés. Vecteurs d'expression et régions de contrôle sont connus de l'homme du métier.

Au cours de la dernière décade, il a été proposé d'utiliser des virus recombinés, comme agents destinés à induire une réponse immunitaire à l'encontre d'organismes pathogènes variés. A cette fin, les adénovirus ou les poxvirus conviennent tout particulièrement. Pour usage dans la présente invention, les poxvirus aviaires, le poxvirus du canari, ou le virus de la vaccine sont tout à fait adaptés. Le virus de la vaccine présente une réaction immunitaire croisée avec le virus de la variole et, de ce fait, a été utilisé comme agent vaccinal anti-variolique depuis le 19e siècle. Au début des années 80, la variole a été considérée comme éradiquée de la surface du globe et l'Organisation Mondiale de la Santé a, en conséquence, jugé préférable d'arrêter de vacciner contre la variole. Le virus de la vaccine est donc maintenant disponible pour mettre en oeuvre des vaccins comprenant un virus de la vaccine dont le génome a été modifié de manière à exprimer des gènes hétérologues codant pour des déterminants antigéniques spécifiques d'un organisme vecteur d'une maladie autre que la variole.

C'est pourquoi l'agent thérapeutique d'une composition pharmaceutique selon l'invention peut être, de manière alternative, un virus dans le génome duquel est inséré un fragment d'ADN codant pour un polypeptide reconnu par l'anticorps H23.

Dans ce cas, le polypeptide, à titre préférentiel, comprend une séquence répétée n fois, n étant un nombre de 1 à 80 ; et de formule (I) : Pro-Gly-Ser-Thr-Ala-Pro-X₁-Ala-His-Gly-Val-Thr-Ser-Ala-Pro-Asp-Y-Arg-Pro-X₂, dans laquelle X₁ et X₂ sont indépendamment Pro ou Ala et Y est Thr ou Asn.

Encore plus préférentiellement, ce polypeptide comprend la séquence de formule (I) répétée n fois et dont la séquence complète présente un degré d'homologie d'au moins 80 % avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante un nombre de 1 à 80, et avantageusement 2, 3 ou 4.

Ce polypeptide peut encore avoir pour séquence (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n étant un nombre de 1 à 80, de préférence n étant 2, 3 ou 4.

Ce type de composition pharmaceutique présente l'avantage d'une production bon marché et d'une grande stabilité dans des conditions d'environnement variées. En particulier, les conditions de conservation ne sont pas contraignantes.

Les conditions générales d'obtention d'un virus de la vaccine capable d'exprimer un bloc d'expression d'une protéine hétérologue sont décrites dans le brevet européen EP 83 286 dont le contenu est ici incorporé par référence. Ces conditions sont applicables aux autres virus acceptables comme vecteurs dans la mesure où ces derniers possèdent au moins une region génomique non-essentielle dans laquelle un bloc d'expression peut être inséré.

Un virus de la vaccine dans le génome duquel est inséré un fragment d'ADN codant pour un polypeptide reconnu par l'anticorps H23 peut être aussi utilisé comme vecteur d'expression particulier en vue de produire ledit polypeptide en culture de cellules de mammifère, tel que précédemment indiqué.

Un polypeptide reconnu par l'anticorps H23 ou un virus dans le génome duquel est inséré un fragment d'ADN codant pour ledit polypeptide présente une activité anti tumorale in vivo dans le test suivant : on traite par deux fois, à dix jours d'intervalle entre les deux traitements, des souris de la lignée C3H ou des rats de la lignée Fisher, âgés de 4 à 5 semaines, avec soit, entre 10 et 500µg d'un polypeptide reconnu par l'anticorps H23 ou soit, entre 10⁷ et 10⁸ pfu (unités formant plaque) d'un virus dans le génome duquel est inséré un fragment d'ADN codant pour ledit polypeptide. Lorsque l'on utilise un polypeptide, le traitement s'effectue de préférence par injection sous-cutanée. Une scarification de la queue est préférée dans le cas d'un virus. Quinze jours après le premier traitement, on injecte de manière sous-cutanée environ 10⁴ à 10⁷ cellules tumorales syngéniques exprimant H23-ETA, qui ont été cultivées in vitro traitées à la trypsine, lavées et resuspendues en tampon PBS (phosphate buffered saline) sous un volume d'environ 100 µl. En parallèle, on soumet de même des animaux non traités à une attaque tumorale identique. Environ 20 jours après l'injection des cellules, la taille des tumeurs sous-cutanées est plus petite chez les animaux traités par un polypeptide ou un virus que chez des animaux non traités.

Un polypeptide reconnu par l'anticorps H23 ou un virus dans le génome duquel est inséré un fragment d'ADN codant pour ledit polypeptide est de ce fait utile en vue de traiter ou de prévenir un état cancéreux, plus particulièrement une tumeur de type carcinome (tumeur développée par des cellules épithéliales), par exemple une tumeur mammaire.

Pour ces prescriptions, le dosage approprié varie en fonction, par exemple du polypeptide ou du virus employé, de l'individu traité, du mode d'administration, de l'utilisation à titre de vaccin ou de traitement, et de la nature et de la sévérité de l'état tumoral qui est traité. Cependant, en général, des résultats de vaccination satisfaisants chez des mammifères, par exemple des humains, sont indiqués comme pouvant être obtenus avec un virus dans le génome duquel est inséré un fragment d'ADN codant pour ledit polypeptide, à un dosage unique ou répété une ou deux fois à environ 1 à 3 semaines d'intervalle, d'environ 10⁴ pfu/kg à environ 10⁸pfu/kg du poids corporel du mammifère.

Une composition pharmaceutique selon l'invention peut être administrée par n'importe quelle voie conventionnelle, en particulier par voie sous-cutanée, par exemple sous forme de solution ou de suspension injectable. A titre de vaccin, une composition selon l'invention peut être administrée selon les modes conventionnellement pratiqués pour les vaccins déjà connus, par exemple en dose unique ou répétée une ou plusieurs fois après un certain délai d'intervalle. Lorsqu'une composition selon l'invention est en usage dans le traitement curatif d'un cancer, elle peut être administrée fréquemment pendant une période suffisante pour que le traitement soit efficace. Une telle composition peut être injectée avantageusement de manière intra-tumorale.

Une composition pharmaceutique selon l'invention peut être préparée selon les techniques conventionnelles. Lorsque l'agent thérapeutique est un virus de la vaccine, ce virus est de préférence sous forme vivante atténuée. Des souches virales atténuées sont disponibles à ce jour; par exemple, la souche Copenhagen thymidine kinase négative. Pour obtenir les virus recombinants nécessaires pour mettre en oeuvre une composition selon l'invention, il suffit d'utiliser une telle souche. Enfin un virus recombinant peut être atténué par un traitement chimique approprié connu de l'homme du métier.

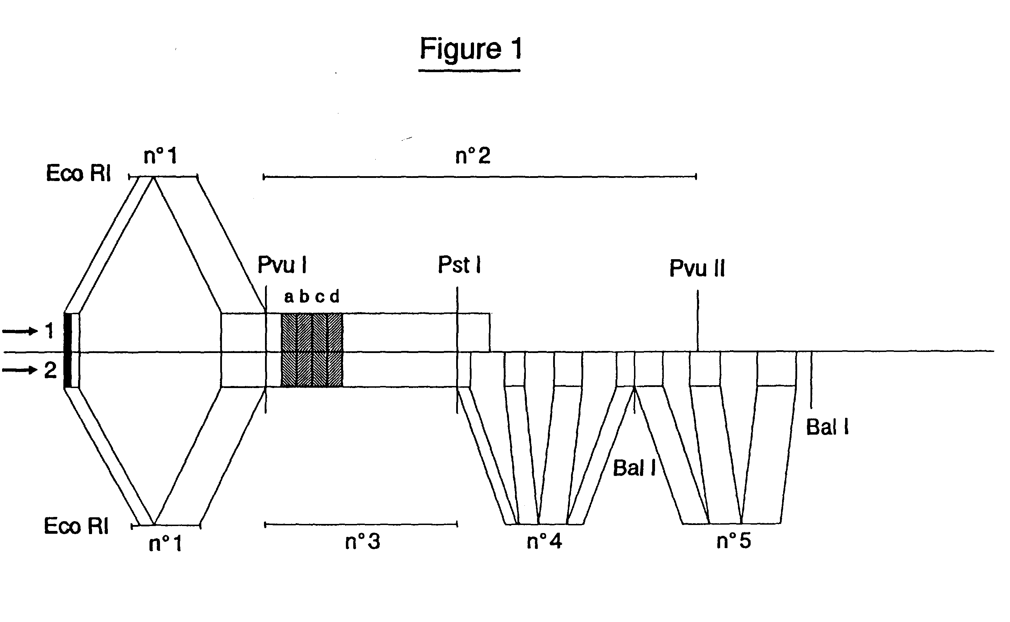

L'invention est illustrée ci-après, avec pour référence la Figure 1.

La Figure 1 représente de manière schématique un fragment d'ADN génomique codant pour la forme sécrétée de H23-ETA (→1) ou pour la forme transmembranaire de H23-ETA (→2), Les blocs et les vides symbolisent respectivement les exons et les introns. Le fond noir correspond à la séquence signal et le fond hachuré signifie les séquences répétées (au nombre de 4: a, b, c et d). Les fragments d'ADN n° 1 et 2 sont utilisés pour la construction d'un fragment complet codant pour la forme sécrétée de H23-ETA tandis que les fragments n° 3 à 5 sont utilisés pour construire un fragment complet codant pour la forme transmembranaire de H23-ETA. Les sites de restriction indiqués dans cette figure se retrouvent de même dans les IS n° 1 et 2.

### Exemple 1

Des fragments d'ADN complémentaires et génomiques codant pour des portions de H23 sont isolés selon la procédure décrite dans Wreschner et al, Eur. J. Biochem. (1990) 189 : 463. Ces fragments sont utilisés par la suite pour reconstituer un fragment d'ADN codant pour l'antigène H23-ETA complet sous sa forme sécrétée ou transmembranaire.

Les constructions plasmidiques sont décrites ci-dessous, en référence à la Figure 1.

### A. Préparation d'un virus de la vaccine capable de promouvoir la synthèse de la forme sécrétée de H23-ETA.

Un fragment d'ADN complémentaire EcoRI-PvuII (n° 1) est introduit entre les sites EcoRI et PvuII de la région d'insertion multiple du vecteur pPolyII décrit dans Lathe et al, Gene (1987) 57 : 193 pour donner le plasmide pETA-5'. Un fragment d'ADN génomique PvuII (n° 2), comportant 4 unités répétées, est introduit dans le site PvuII de la région d'insertion multiple de pETA-5', en aval du fragment n° 1 et en orientation appropriée. Dans les unités répétées a, b, c et d, les codons xxx₁ et xxx₂ sont respectivement CCA (Pro) et CCC (Pro), CCA et CCC, GCA (Ala) et GCC, CCA et GCC. De même le codon yyy est ACC (Thr) dans les unités répétées a, b et c; le codon yyy est AAC (Asn) dans l'unité d.

Un fragment BamHl-SalI codant pour la forme sécrétée complète de H23-ETA est excisé du plasmide obtenu en dernier lieu. Puis, ce fragment est inséré entre les sites BamHI et Sali du vecteur de transfert ptg194-poly décrit dans Kieny et al, Bio/Technology, (1986) 4:790, en aval du promoteur du virus de la vaccine E7.5k et à l'intérieur du gène du virus de la vaccine codant pour la thymidine kinase.

Le vecteur de transfert obtenu au paragraphe précédent est utilisé par la suite pour transférer le bloc d'expression de la forme sécrétée de H23-ETA dans le génome du virus de la vaccine, souche Copenhagen, selon la méthode décrite dans Kieny et al, Nature (1984) 312 : 163. On obtient ainsi le virus de la vaccine VV-ETA-S.

### B. Préparation d'un virus de la vaccine capable de promouvoir la synthèse de la forme transmembranaire de H23-ETA.

Un fragment d'ADN génomique PvuII-PstI (n° 3), comportant 4 unités répétées, est introduit entre les sites PvuII et PstI de la région d'insertion multiple de pETA-5', en aval du fragment n° 1 et en orientation appropriée. Dans les unités répétées a, b, c et d, les codons xxx₁ et xxx₂ sont respectivement CCA (Pro) et CCC (Pro), CCA et CCC, GCA (Ala) et GCC, CCA et GCC. De même le codon yyy est ACC (Thr) dans les unités répétées a, b et c; le codon yyy est AAC (Asn) dans l'unité d.

Un fragment EcoRI-PstI correspondant aux fragments clonés est excisé du dernier plasmide obtenu. L'extrémité cohésive EcoRI est transformée en extrémité franche par traitement avec la polymérase klenow. Puis, ce fragment est introduit entre le site XhoI, préalablement traité par la polymérase klenow, et le site PstI de la région d'insertion mutiple du vecteur pPolyII-Sfi/Not-14 décrit dans Lathe et al, supra, pour donner le plasmide pETA-T-5'.

Un fragment d'ADN complémentaire PstI-BalI (n° 4) est introduit entre les sites PstI et BalI de pETA-T-5'. Puis, un fragment d'ADN complémentaire BalI-BalI (n° 5) est inséré dans le site BalI du plasmide obtenu en dernier lieu.

Un fragment BglII-SstI codant pour la forme transmembranaire complète de H23-ETA est excisé du plasmide obtenu au paragraphe précédent; puis, il est introduit entre les sites BamHI et SstI du vecteur de transfert ptgl86-poly décrit dans Kieny et al, (1986) supra, en aval du promoteur du virus de la vaccine E7.5k et à l'intérieur du gène du virus de la vaccine codant pour la thymidine kinase.

Le vecteur de transfert obtenu au paragraphe précédent est utilisé par la suite pour transférer le bloc d'expression de la forme transmembranaire de H23-ETA dans le génome du virus de la vaccine, souche Copenhagen (VV-O), selon la méthode décrite dans Kieny et al, 1984, supra. On obtient ainsi le virus de la vaccine VV-ETA-T.

### Exemple 2: Préparation des stocks de virus.

Les stocks de virus purifiés sont préparés sur cellules BHK-21. Les cellules BHK-21 sont infectées par les virus recombinants VV-ETA-S et VV-ETA-T (0,1 pfu/cellule) pendant 48 heures. Après ce temps, les cultures sont congelées à -20°C, puis décongelées à température ambiante. Après destruction des parois cellulaires par 3 traitements successifs au "potter" dans un tampon hypotonique, les protéines solubles du surnageant sont chargées sur un coussin de saccharose 36 % (p/v) et centrifugées (SW 28 Beckman, 1h, 14 K). Le culot contenant le virus est repris en solution dans du Tris/HCl 10 mM pH8 et déposé sur un gradient linéaire (20-40 %) de saccharose. Après centrifugation (SW 28, 40 min, 14 K), la bande opalescente contenant le virus est reprise à l'aide d'une seringue et concentrée par centrifugation (SW 28, 20 K, 1h). Le virus est enfin repris dans un petit volume de Tris/Hcl 10 mM pH8 de façon à obtenir un stock viral titrant 10¹⁰ pfu/ml environ.

### Exemple 3: Lignées cellulaires tumorales exprimant H23-ETA.

### A. Construction des plasmides eucaryotes capable de promouvoir l'expression de H23-ETA.

Un fragment d'ADN BamHI-SalI, codant pour la forme sécrétée de H23-ETA est excisé du plasmide obtenu dans l'exemple 1A, premier paragraphe. Puis, il est réintroduit entre les sites BamHI et SalI de la région d'insertion multiple du plasmide pHMG décrit dans Gautier et al, Nucl. Acid Res., (1989) 17 (20): 83, de manière à être placé sous le contrôle du promoteur du gène de la 3-hydroxy-3-méthyl-glutaryl-coenzyme-A-réductase (HMGCR), en aval de la séquence signal du SV40 polyA. On obtient ainsi le plasmide pHMG-ETA-S.

De même, le plasmide pHMG-ETA-T est construit, de manière similaire, par insertion d'un fragment d'ADN BamHI-EcoRV issu du plasmide obtenu dans l'Exemple 1 B, paragraphe 2.

### B. Préparation des lignées cellulaires.

Des cellules de la lignée cellulaire tumorale FR3T3-ras-1 obtenue à partir de fibroblastes de rats Fisher par Matriceau et al, EMBO J. (1985) 4 : 1435 et de cellules de la lignée de carcinome mammaire de souris MM5t issue des souris C3H, sont cotransfectées (i) par pHMG-ETA-S et le plasmide pAG60 décrit dans Colbere-Garapin et al, J. Mol. Biol. (1981) 150 : 1 qui comporte un gène de résistance à la Généticine (G418) ou (ii) par pHMG-ETA-T et pAG60. Pour effectuer la transfection, on utilise la méthode de précipitation au phosphate de calcium de Graham et al, Virology (1973) 52 : 456 modifiée par Wigler et al, Cell (1978) 14: 725.

Les clones transfectés sont selectionnés en présence de 500 µl/ml de G418 et par la suite cultivés. La sélection des clones exprimant H23-ETA s'effectue par marquage des cellules à la péroxidase après réaction avec l'anticorps H23. Des lignées cellulaires à l'état pur sont obtenues par la méthode des dilutions limites et l'expression de H23-ETA est contrôlée.
Les lignées cellulaires sont prénommées comme suit :
FR3T3-ras-1 (pAG60/pHMG-ETA-S) : F-S
FR3T3-ras-1 (pAG60/pHMG-ETA-T) : F-T
FR3T3-ras-1 (pAG60/pHMG) : F-C
MM5tC3H (pAG60/pHMG-ETA-S) : M-S
MM5tC3H (pAG60/pHMG-ETA-S) : M-T
MM5tC3H (pAG60/pHMG-ETA-S) : M-C

### Exemple 4: Mise en évidence de l'effet vaccinal de H23-ETA.

Des rats mâles et femelles de la lignée IOPS Fisher et des souris femelles de la lignée C3H ages de 4 à 5 semaines sont immunisés de la façon suivante : une préparation virale purifiée de VV-ETA-S, VV-ETA-T ou VV-O est administrée aux animaux, par scarification de la queue, sous un volume de 10 µl correspondant à environ 2.10⁷ pfu. Ce traitement est répété 10 jours après.

Les lignées tumorales F-S, F-T, F-C, M-S, M-T et M-C sont cultivées dans un milieu Dulbecco modifié (Gibco) supplémenté avec 10% de sérum de veau foetal, 100 unités de pénicilline et 100 µg/ml de streptomycine. Les cultures sont ensuite traitées à la trypsine, lavées, et suspendues en tampon PBS (phosphate buffered saline).

14 jours après la première étape d'immunisation, 2.10⁴ cellules F-C, 4.10⁴ cellules F-S, 1.5 10⁵ cellules F-T ou 2.10⁶ M-C, M-S ou M-T sont injectées à un animal de manière sous-cutanée, sous un volume de 100 µl.

L'apparition des tumeurs sous-cutanées est contrôlée quotidiennement. Le diamètre des tumeurs est mesuré en deux dimensions. Les données complètes de l'expérience et les résultats sont présentés dans le tableau I ci-dessous :

**Tableau I**

| Animal | Virus | Cellules tumorales | Nombre d'animaux présentant un nodule tumoral par rapport au nombre total d'animaux traités | Diamètre moyen des nodules tumoraux (en mm) mesuré x jours après l'injection des cellules | Pourcentage d'animaux exempts de tumeurs |
|---|---|---|---|---|---|
| Rats mâles de la lignée Fisher | | F-C | 4/4 | 31 (20 jours) | 0 |
| | | F-S | 3/4 | 25 (25 jours) | 25 |
| | | F-T | 3/6 | 25 (30 jours) | 50 |
| | VV.ETA-S | F-C | 8/8 | 40 (20 jours) | 0 |
| | | F-S | 3/8 | 7,5 (25 jours) | 62,5 |
| | | F-T | 1/8 | 0,87 (30 jours) | 87,5 |
| | VV-ETA-T | F-C | 8/8 | 32 (20 jours) | 0 |
| | | F-S | 1/8 | 0,38 (25 jours) | 87,5 |
| | | F-T | 0/8 | 0 (30 jours) | 100 |
| | | F-S | 10/10 | 11,2 (20 jours) | 0 |
| | | F-T | 10/10 | 25 (20 jours) | 0 |
| | VV-ETA-S | F-S | 9/10 | 16 (20 jours) | 10 |
| | | F-T | 9/10 | 30 (20 jours) | 10 |
| | VV-ETA-T | F-S | 5/10 | 1,7 (20 jours) | 50 |
| | | F-T | 5/10 | 2,8 (20 jours) | 50 |
| Rats femelles de la lignée Fisher | VV-0 | F-S | 10/10 | 19,6 (20 jours) | 0 |
| | | F-T | 10/10 | 28 (20 jours) | 0 |
| | VV-ETA-S | F-S | 8/10 | 10,6 (20 jours) | 20 |
| | | F-T | 9/9 | 33,8 (20 jours) | 0 |
| | VV-ETA-T | F-S | 5/10 | 0,1 (25 jours) | 50 |
| | | F-T | 1/10 | | 90 |

Le tableau I montre que, lorsque les animaux sont soumis à une infection par F-S ou F-T, la fréquence d'apparition des tumeurs dans un lot d'animaux préalablement traités à l'aide du virus de la vaccine VV-ETA-S ou VV-ETA-T est moins élevée que dans les lots d'animaux non traités ou traités avec un virus de la vaccine VV-O. D'autre part, la taille des nodules tumoraux qui apparaissent chez des animaux préalablement traités avec VV-ETA-S ou VV-ETA-T est beaucoup plus petite que celle des nodules tumoraux observés chez les animaux non traités ou traités avec VV-O.

L'immunisation à l'aide de VV-ETA-S ou VV-ETA-T n'est efficace que dans le cas des tumeurs induites par des cellules exprimant la forme sécrétée ou transmembranaire de H23-ETA. L'effet vaccinal des virus est donc bien spécifique.

Enfin, il semble que l'effet vaccinal de VV-ETA-T soit supérieur à celui de VV-ETA-S, quelle que soit la forme de H23-ETA exprimée par les cellules induisant les tumeurs.

### Exemple 5: Mise en évidence de l'effet curatif de H23-ETA.

Des rats de la lignée Fisher sont infectés par des cellules tumorales, tel que décrit dans l'Exemple 4. Dès l'apparition des tumeurs (10 à 15 jours après), on procède au traitement à l'aide des préparations virales, tel que décrit dans l'Exemple 4.
Les données et résultats de l'expérience sont présentés dans le tableau II ci-dessous :

**Tableau II**

| Virus | Cellules tumorales | Nombre d'animaux présentant un nodule tumoral par rapport au nombre total d'animaux traités | | Diamètre moyen des des tumeurs mesuré (en mm) | |
|---|---|---|---|---|---|
| | | 25 jours après l'injection | 50 jours après l'injection | 25 jours après l'injection | 50 jours après l'injection |
| VV-0 | F-S | 10/10 | 10/10 | 27.8 | tous morts |
| | F-T | 10/10 | 10/10 | 27.7 | tous morts |
| VV-ETA-S | F-S | 10/10 | 10/10 | 31.5 | tous morts |
| | F-T | 9/10 | 7/10 | 15.5 | 8.5 |
| VV-ETA-T | F-S | 9/10 | 10/10 | 26.8 | 50.2 |
| | F-T | 7/10 | 7/10 | 11.6 | 9.4 |

Le Tableau II montre que le traitement d'une infection par VV-ETA-S ou VV-ETA-T a une incidence favorable sur la fréquence d'apparition et la taille des tumeurs par rapport au test contrôle. D'autre part, il semble que VV-ETA-T soit plus efficace que VV-ETA-S.

### IDENTIFICATEUR DE SEQUENCE N° 1

- **Objet:**: La forme transmembranaire de l'antigène H23-ETA
- **Type de séquence:**: Séquence d'un fragment d'ADN et la séquence d'acides aminés correspondante
- **Type de molécule:**: ADN complémentaire
- **Origine:**: Lignée de carcinome mammaire T47D
**Caractèristiques du fragment d'ADN complet**:
Fragment EcoRI-BalI
Séquence codante: du nucléotide 58 au nucléotide 1362+(60xn)
**Caractèristiques de la séquence en acides aminés**:
- Peptide signal:: de l'a.a. -21 à l'a.a. -1
- Forme mature:: de l'a.a. 1 à l'a.a. 414*, * signifiant [+(20xn)] dans lequel n est un nombre de 1 à 80
- Séquence répétée:: Telle que montrée encadrée ci-dessous, dans laquelle X₁ et X₂ sont indépendamment Pro ou Ala et Y est Thr ou Asn

GAATTCCCTG GCTGCTTGAA TCTGTTCTGC CCCCTCCCCA CCCATTTCAC 50

CACCACC ATG ACA CCG GGC ACC CAG TCT CCT TTC TTC CTG 90
Met Thr Pro Gly Thr Gln Ser Pro Phe Phe Leu
-21 -20 -15

CTG CTG CTC CTC ACA GTG CTT ACA GTT GTT ACA GGT TCT 129
Leu Leu Leu Leu Thr Val Leu Thr Val Val Thr Gly Ser
-10 -5 -1 1

GGT CAT GCA AGC TCT ACC CCA GGT GGA GAA AAG GAG ACT 168
Gly His Ala Ser Ser Thr Pro Gly Gly Glu Lys Glu Thr
5 10 15

TCG GCT ACC CAG AGA AGT TCA GTG CCC AGC TCT ACT GAG 207
Ser Ala Thr Gln Arg Ser Ser Val Pro Ser Ser Thr Glu
20 25

AAG AAT GCT GTG AGT ATG ACC AGC AGC GTA CTC TCC AGC 246
Lys Asn Ala Val Ser Met Thr Ser Ser Val Leu Ser Ser
30 35 40

CAC AGC CCC GGT TCA GGC TCC TCC ACC ACT CAG GGA CAG 285
His Ser Pro Gly Ser Gly Ser Ser Thr Thr Gln Gly Gln
45 50 55

GAT GTC ACT CTG GCC CCG GCC ACG GAA CCA GCT TCA GGT 324
Asp Val Thr Leu Ala Pro Ala Thr Glu Pro Ala Ser Gly
60 65

PvuII
TCA GCT GCC ACC TGG GGA CAG GAT GTC ACC TCG GTC CCA 363
Ser Ala Ala Thr Trp Gly Gln Asp Val Thr Ser Val Pro
70 75 80

GTC ACC AGG CCA GCC CTG GGC TCC ACC ACC CCG CCA GCC 402
Val Thr Arg Pro Ala Leu Gly Ser Thr Thr Pro Pro Ala
85 90

CAC GAT GTC ACC TCA GCC CCG GAC AAC AAG CCA GCC|CCG
His Asp Val Thr Ser Ala Pro Asp Asn Lys Pro Ala|Pro
95 100 105

GGC TCC ACC GCC CCC xxx GCC CAC GGT GTC ACC TCG GCC
Gly Ser Thr Ala Pro X1 Ala His Gly Val Thr Ser Ala

CCG GAC yyy AGG CCG xxx|TTG GGC TCC ACC GCC CCT CCA 459 + (60xn)
Pro Asp Y Arg Pro X2|Leu Gly Ser Thr Ala Pro Pro
n 110*

GTC CAC AAT GTC ACC TCG GCC TCA GGC TCT GCA TCA GGC 498 + (60xn)
Val His Asn Val Thr Ser Ala Ser Gly Ser Ala Ser Gly
115* 120* 125*

TCA GCT TCT ACT CTG GTG CAC AAC GGC ACC TCT GCC AGG 537 + (60xn)
Ser Ala Ser Thr Leu Val His Asn Gly Thr Ser Ala Arg
130* 135*

GCT ACC ACA ACC CCA GCC AGC AAG AGC ACT CCA CCC AGC 576 + (60xn)
Ala Thr Thr Thr Pro Ala Ser Lys Ser Thr Pro Phe Ser
140* 145* 150*

ATT CCC AGC CAC CAC TCT GAT ACT CCT ACC ACC CTT GCC 615 + (60xn)
Ile Pro Ser His His Ser Asp Thr Pro Thr Thr Leu Ala
155* 160* 165*

AGC CAT AGC ACC AAG ACT GAT GCC AGT AGC ACT CAC CAT 654 + (60xn)
Ser His Ser Thr Lys Thr Asp Ala Ser Ser Thr His His
170* 175*

AGC ACG GTA CCT CCT CTC ACC TCC TCC AAT CAC AGC ACT 693 + (60xn)
Ser Thr Val Pro Pro Leu Thr Ser Ser Asn His Ser Thr
180* 185* 190*

TCT CCC CAG TTG TCT ACT GGG GTC TCT TTC TTT TTC CTG 732 + (60xn)
Ser Pro Gln Leu Ser Thr Gly Val Ser Phe Phe Phe Leu
195* 200*

TCT TTT CAC ATT TCA AAC CTC CAG TTT AAT TCC TCT CTG 771 + (60xn)
Ser Phe His Ile Ser Asn Leu Gln Phe Asn Ser Ser Leu
205* 210* 215*
PstI
GAA GAT CCC AGC ACC GAC TAC TAC CAA GAG CTG CAG AGA 810 + (60xn)
Glu Asp Pro Ser Thr Asp Tyr Tyr Gln Glu Leu Gln Arg
220* 225* 230*

GAC ATT TCT GAA ATG TTT TTG CAG ATT TAT AAA CAA GGG 849 + (60xn)
Asp Ile Ser Glu Met Phe Leu Gln Ile Tyr Lys Gln Gly
235* 240*

GGT TTT CTG GGC CTC TCC AAT ATT AAG TTC AGG CCA GGA5 888 + (60xn)
Gly Phe Leu Gly Leu Ser Asn Ile Lys Phe Arg Pro Gly
245* 250* 255*

TCT GTG GTG GTA CAA TTG ACT CTG GCC TTC CGA GAA GGT 927 + (60xn)
Ser Val Val Val Gln Leu Thr Leu Ala Phe Arg Glu Gly
260* 265*

ACC ATC AAT GTC CAC GAC GTG GAG ACA CAG TTC AAT CAG 966 + (60xn)
Thr Ile Asn Val His Asp Val Glu Thr Gln Phe Asn Gln
270* 275* 280*

TAT AAA ACG GAA GCA GCC TCT CGA TAT AAC CTG ACG ATC 1005 + (60xn)
Tyr Lys Thr Glu Ala Ala Ser Arg Tyr Asn Leu Thr Ile
285* 290* 295*

TCA GAC GTC AGC GTG AGT CAT GTG CCA TTT CCT TTC TCT 1044 + (60xn)
Ser Asp Val Ser Val Ser His Val Pro Phe Pro Phe Ser
300* 305*

GCC CAG TCT GGG GCT GGG GTG CCA GGC TGG GGC ATC GCG 1083 + (60xn)
Ala Gln Ser Gly Ala Gly Val Pro Gly Trp Gly Ile Ala
310* 315* 320*
BalI
CTG CTG GTG CTG GTC TGT GTT CTG GTT GCG CTG GCC ATT 1122 + (60xn)
Leu Leu Val Leu Val Cys Val Leu Val Ala Leu Ala Ile
325* 330*

GTC TAT CTC ATT GCC TTG GCT GTC TGT CAG TGC CGC CGA 1161 + (60xn)
Val Tyr Leu Ile Ala Leu Ala Val Cys Gln Cys Arg Arg
335* 340* 345*

AAG AAC TAC GGG CAG CTG GAC ATC TTT CCA GCC CGG GAT 1200 + (60xn)
Lys Asn Tyr Gly Gln Leu Asp Ile Phe Pro Ala Arg Asp
350* 355* 360*

ACC TAC CAT CCT ATG AGC GAG TAC CCC ACC TAC CAC ACC 1239 + (60xn)
Thr Tyr His Pro Met Ser Glu Tyr Pro Thr Tyr His Thr
365* 370*

CAT GGG CGC TAT GTG CCC CCT AGC AGT ACC GAT CGT AGC 1278 + (60xn)
His Gly Arg Tyr Val Pro Pro Ser Ser Thr Asp Arg Ser
375* 380* 385*

CCC TAT GAG AAG GTT TCT GCA GGT AAT GGT GGC AGC AGC 1317 + (60xn)
Pro Tyr Glu Lys Val Ser Ala Gly Asn Gly Gly Ser Ser
390* 395*

CTC TCT TAC ACA AAC CCA GCA GTG GCA GCC ACT TCT GCC 1356 + (60xn)
Leu Ser Tyr Thr Asn Pro Ala Val Ala Ala Thr Ser Ala
400* 405* 410*

AAC TTG TAG GGGCACGTCG CCCTCTGAGC TGAGTGG 1392 + (60xn)
Asn Leu Ter

### IDENTIFICATEUR DE SEQUENCE N° 2

- **Objet:**: La forme soluble de l'antigène H23-ETA
- **Type de séquence:**: Séquence d'un fragment d'ADN et la séquence d'acides aminés correspondante
- **Type de molécule:**: ADN complémentaire
- **Origine:**: Lignée de carcinome mammaire T47D
**Caractèristiques du fragment d'ADN complet:**
Fragment EcoRI-PvuII
Séquence codante: du nucléotide 58 au nucléotide 858+(60xn)
**Caractéristiques de la séquence en acides aminés:**
- Peptide signal:: de l'a.a. -21 à l'a.a. -1
- Forme mature:: de l'a.a. 1 à l'a.a. 246*, * signifiant [+(20xn] dans lequel n est un nombre de 1 à 80
- Séquence répétée:: Telle que montrée encadrée ci-dessous, dans laquelle X₁ et X₂ sont indépendamment Pro ou Ala et Y est Thr ou Asn

GAATTCCCTG GCTGCTTGAA TCTGTTCTGC CCCCTCCCCA CCCATTTCAC 50

CACCACC ATG ACA CCG GGC ACC CAG TCT CCT TTC TTC CTG 90
Met Thr Pro Gly Thr Gln Ser Pro Phe Phe Leu
-21 -20 -15

CTG CTG CTC CTC ACA GTG CTT ACA GTT GTT ACA GGT TCT 129
Leu Leu Leu Leu Thr Val Leu Thr Val Val Thr Gly Ser
-10 -5 -1 1

GGT CAT GCA AGC TCT ACC CCA GGT GGA GAA AAG GAG ACT 168
Gly His Ala Ser Ser Thr Pro Gly Gly Glu Lys Glu Thr
5 10 15

TCG GCT ACC CAG AGA AGT TCA GTG CCC AGC TCT ACT GAG 207
Ser Ala Thr Gln Arg Ser Ser Val Pro Ser Ser Thr Glu
20 25

AAG AAT GCT GTG AGT ATG ACC AGC AGC GTA CTC TCC AGC 246
Lys Asn Ala Val Ser Met Thr Ser Ser Val Leu Ser Ser
30 35 40

CAC AGC CCC GGT TCA GGC TCC TCC ACC ACT CAG GGA CAG 285
His Ser Pro Gly Ser Gly Ser Ser Thr Thr Gln Gly Gln
45 50 55

GAT GTC ACT CTG GCC CCG GCC ACG GAA CCA GCT TCA GGT 324
Asp Val Thr Leu Ala Pro Ala Thr Glu Pro Ala Ser Gly
60 65

PvuII
TCA GCT GCC ACC TGG GGA CAG GAT GTC ACC TCG GTC CCA 363
Ser Ala Ala Thr Trp Gly Gln Asp Val Thr Ser Val Pro
70 75 80

GTC ACC AGG CCA GCC CTG GGC TCC ACC ACC CCG CCA GCC 402
Val Thr Arg Pro Ala Leu Gly Ser Thr Thr Pro Pro Ala
85 90

CAC GAT GTC ACC TCA GCC CCG GAC AAC AAG CCA GCC|CCG
His Asp Val Thr Ser Ala Pro Asp Asn Lys Pro Ala|Pro
95 100 105

GGC TCC ACC GCC CCC xxx GCC CAC GGT GTC ACC TCG GCC
Gly Ser Thr Ala Pro X₁ Ala His Gly Val Thr Ser Ala

CCG GAC yyy AGG CCG xxx|TTG GGC TCC ACC GCC CCT CCA 459 + (60xn)
Pro Asp Y Arg Pro X₂|Leu Gly Ser Thr Ala Pro Pro
n 110*

GTC CAC AAT GTC ACC TCG GCC TCA GGC TCT GCA TCA GGC 498 + (60xn)
Val His Asn Val Thr Ser Ala Ser Gly Ser Ala Ser Gly
115* 120* 125*

TCA GCT TCT ACT CTG GTG CAC AAC GGC ACC TCT GCC AGG 537 + (60xn)
Ser Ala Ser Thr Leu Val His Asn Gly Thr Ser Ala Arg
130* 135*

GCT ACC ACA ACC CCA GCC AGC AAG AGC ACT CCA TTC TCA 576 + (60xn)
Ala Thr Thr Thr Pro Ala Ser Lys Ser Thr Pro Phe Ser
140* 145* 150*

ATT CCC AGC CAC CAC TCT GAT ACT CCT ACC ACC CTT GCC 615 + (60xn)
Ile Pro Ser His His Ser Asp Thr Pro Thr Thr Leu Ala
155* 160* 165*

AGC CAT AGC ACC AAG ACT GAT GCC AGT AGC ACT CAC CAT 654 + (60xn)
Ser His Ser Thr Lys Thr Asp Ala Ser Ser Thr His His

170*                    175*
AGC ACG GTA CCT CCT CTC ACC TCC TCC AAT CAC AGC ACT          693 + (60xn)
Ser Thr Val Pro Pro Leu Thr Ser Ser Asn His Ser Thr
    180*                185*                190*

TCT CCC CAG TTG TCT ACT GGG GTC TCT TTC TTT TTC CTG          732 + (60xn)
Ser Pro Gln Leu Ser Thr Gly Val Ser Phe Phe Phe Leu
            195*                200*

TCT TTT CAC ATT TCA AAC CTC CAG TTT AAT TCC TCT CTG          771 + (60xn)
Ser Phe His Ile Ser Asn Leu Gln Phe Asn Ser Ser Leu
205*                210*                215*

GAA GAT CCC AGC ACC GAC TAC TAC CAA GAG CTG CAG AGA          810 + (60xn)
Glu Asp Pro Ser Thr Asp Tyr Tyr Gln Glu Leu Gln Arg
        220*                225*                230*

GAC ATT TCT GAA ATG GTG AGT ATC GGC CTT TCC TTC CCC          849 + (60xn)
Asp Ile Ser Glu Met Val Ser Ile Gly Leu Ser Phe Pro
                235*                240*

ATG CTC CCC TGA AGCAGCCATC AGAACTGTCC ACACCCTTTG          891 + (60xn)
Met Leu Pro Ter
    245*

CATCAAGCCT GAGTCCTTTC CCTCTCACCC CAGTTTTTGC AGATTTATAA          941 + (60xn)
ACAAGGGGGT TTTCTGGGCC TCTCCAATAT TAAGTTCAGG TACAGTTCTG          991 + (60xn)
GGTGTGGACC CAGTGTGGTG GTTGGAGGGT TGGGTGGTGG TCATGACCGT          1041 + (60xn)
AGGAGGGACT GGTCGCACTT AAGGTTGGGG GAAGAGTCGT GAGCCAGAGC          1091 + (60xn)
TGGGACCCGT GGCTGAAGTG CCCATTTCCC TGTGACCAGG CCAGGATCTG          1141 + (60xn)
TGGTGGTACA ATTGACTCTG GCCTTCCGAG AAGGTACCAT CAATGTCCAC          1191 + (60xn)
GACGTGGAGA CACAGTTCAA TCAGTATAAA ACGGAAGCAG CCTCTCGATA          1241 + (60xn)
TAACCTGACG ATCTCAGACG TCAGCGGTGA GGCTACTTCC CTGGCTGCAG          1291 + (60xn)
CCCAGCACCA TGCCGGGGCC CTCTCCTTCC AGTGCCTGGG TCCCCGCTCT          1341 + (60xn)
TTCCTTAGTG CTGGCAGCGG GAGGGGCGCC TCCTCTGGGA GACTGCCCTG          1391 + (60xn)
ACCACTGCTT TTCCTTTTAG TGAGTCATGT GCCATTTCCT TTCTCTGCCC          1441 + (60xn)
AGTCTGGGGC TGGGGTGCCA GGCTGGGGCA TCGCGCTGCT GGTGCTGGTC          1491 + (60xn)
TGTGTTCTGG TTGCGCTGGC CATTGTCTAT CTCATTGCCT TGGTGAGTGC          1541 + (60xn)
AGTCCCTGGC CCTGATCAGA GCCCCCCGGT AGAAGGCACT CCATGGCCTG          1591 + (60xn)
CCATAACCTC CTATCTCCCC AGGCTGTCTG TCAGTGCCGC CGAAAGAACT          1641 + (60xn)
ACGGGCAG          1649 + (60xn)

## Revendications (Revendications pour l'(les) Etat(s) contractant(s) suivant(s): AT, BE, CH, DE, DK, FR, GB, IT, LI, LU, NL, SE)

1. Une composition pharmaceutique pour traiter ou prévenir une tumeur maligne qui comprend, à titre d'agent thérapeutique, un polypeptide reconnu par l'anticorps H23 en association avec un diluent ou un support acceptable d'un point de vue pharmaceutique et dans laquelle le polypeptide reconnu par l'anticorps H23 comprend une. séquence répétée n fois et de formule (I) : Pro-Gly-Ser-Thr-Ala-Pro-X₁-Ala-His-Gly-Val-Thr-Ser-Ala-Pro-Asp-Y-Arg-Pro-X₂, dans laquelle X₁ et X₂ sont indépendamment Pro ou Ala et Y est Thr ou Asn, à l'exclusion de la séquence dans laquelle X₁, X₂ et Y représentent respectivement Pro, Ala et Thr, ledit polypeptide ayant une séquence complète présentant un degré d'homologie d'au moins 80 % avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante un nombre de 1 à 80.

2. Une composition selon la revendication 1, dans laquelle le polypeptide reconnu par l'anticorps H23 est un polypeptide comprenant la séquence de formule (I) répétée n fois dont la séquence complète présente un degré d'homologie d'au moins 80 % avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante, 2, 3 ou 4.

3. Une composition selon la revendication 1, dans laquelle le polypeptide reconnu par l'anticorps H23 a pour séquence (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; n étant un nombre de 1 à 80.

4. Une composition selon la revendication 3, dans laquelle le polypeptide reconnu par l'anticorps H23 a pour séquence (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; n étant 2, 3 ou 4.

5. Une composition pharmaceutique pour traiter ou prévenir une tumeur maligne, qui comprend, à titre d'agent thérapeutique, un virus dans le génome duquel est inséré un fragment d'ADN codant pour un polypeptide reconnu par l'anticorps H23, ledit polypeptide comprenant une séquence répétée n fois de formule (I) : Pro-Gly-Ser-Thr-Ala-Pro-X₁-Ala-His-Gly-Val-Thr-Ser-Ala-Pro-Asp-Y-Arg-Pro-X₂, dans laquelle X₁ et X₂ sont indépendamment Pro ou Ala et Y est Thr ou Asn et dont la séquence complète présente un degré d'homologie d'au moins 80 % avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante un nombre de 1 à 80, ledit fragment d'ADN étant placé sous le contrôle de signaux de transcription et de traduction appropriés.

6. Une composition selon la revendication 5, qui comprend un virus dans le génome duquel est inséré un fragment d'ADN codant pour un polypeptide reconnu par l'anticorps H23, comprenant la séquence de formule (I) répétée n fois et dont la séquence complète présente un degré d'homologie d'au moins 80 % avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante 2, 3 ou 4.

7. Une composition selon la revendication 5, qui comprend un virus dans le génome duquel est inséré un fragment d'ADN codant pour un polypeptide reconnu par l'anticorps H23, ayant pour séquence (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n étant un nombre de 1 à 80.

8. Une composition selon la revendication 7, qui comprend un virus dans le génome duquel est inséré un fragment d'ADN codant pour un polypeptide reconnu par l'anticorps H23, ayant pour séquence (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n étant 2, 3 ou 4.

9. Une composition selon n'importe laquelle des revendications 5 à 8, dans laquelle le virus dans le génome duquel est inséré un fragment d'ADN codant pour un polypeptide reconnu par l'anticorps H23 est un poxvirus.

10. Une composition selon la revendication 9, dans laquelle le poxvirus dans le génome duquel est inséré un fragment d'ADN codant pour un polypeptide reconnu par l'anticorps H23 est le virus de la vaccine.

11. Une composition selon n'importe laquelle des revendications 5 à 8, dans laquelle le virus dans le génome duquel est inséré un fragment d'ADN codant pour un polypeptide reconnu par l'anticoprs H23 est un adénovirus.

## Revendications (Revendications pour l'(les) Etat(s) contractant(s) suivant(s): ES, GR)

1. Utilisation d'une composition pharmaceutique pour la préparation d'un médicament destiné au traitement ou à la prévention d'une tumeur maligne, ladite composition comprenant, à titre d'agent thérapeutique, un polypeptide reconnu par l'anticorps H23 en association avec un diluent ou un support acceptable d'un point de vue pharmaceutique et dans laquelle le polypeptide reconnu par l'anticorps H23 comprend une séquence répétée n fois et de formule (I) : Pro-Gly-Ser-Thr-Ala-Pro-X₁-Ala-His-Gly-Val-Thr-Ser-Ala-Pro-Asp-Y-Arg-Pro-X₂, dans laquelle X₁ et X₂ sont indépendamment Pro ou Ala et Y est Thr ou Asn, à l'exclusion de la séquence dans laquelle X₁, X₂ et Y représentent respectivement Pro, Ala et Thr, ledit polypeptide ayant une séquence complète présentant un degré d'homologie d'au moins 80% avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante un nombre de 1 à 80.

2. Utilisation d'une composition pharmaceutique selon la revendication 1, dans laquelle le polypeptide reconnu par l'anticorps H23 est un polypeptide comprenant la séquence de formule (I) répétée n fois dont la séquence complète présente un degré d'homologie d'au moins 80% avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante, 2, 3 ou 4.

3. Utilisation d'une composition selon la revendication 1, dans laquelle le polypeptide reconnu par l'anticorps H23 a pour séquence (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; n étant un nombre de 1 à 80.

4. Utilisation d'une composition selon la revendication 3, dans laquelle le polypeptide reconnu par l'anticorps H23 a pour séquence (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; n étant 2, 3 ou 4.

5. Utilisation d'une composition pharmaceutique pour la préparation d'un médicament destiné au traitement ou à la prévention d'une tumeur maligne, ladite composition comprenant, à titre d'agent thérapeutique, un virus dans le génome duquel est inséré un fragment d'ADN codant pour un polypeptide reconnu par l'anticorps H23, ledit polypeptide comprenant une séquence répétée n fois et de formule (I) : Pro-Gly-Ser-Thr-Ala-Pro-X₁-Ala-His-Gly-Val-Thr-Ser-Ala-Pro-Asp-Y-Arg-Pro-X₂, dans laquelle X₁ et X₂ sont indépendamment Pro ou Ala et Y est Thr ou Asn et dont la séquence complète présente un degré d'homologie d'au moins 80 % avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante un nombre de 1 à 80, ledit fragment d'ADN étant placé sous le contrôle de signaux de transcription et de traduction appropriés.

6. Utilisation d'une composition selon la revendication 5, qui comprend un virus dans le génome duquel est inséré un fragment d'ADN codant pour un polypeptide reconnu par l'anticorps H23, comprenant la séquence de formule (I) répétée n fois et dont la séquence complète présente un degré d'homologie d'au moins 80 % avec (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n relatif à la séquence de formule (I) dudit polypeptide d'une part, et celui relatif à la séquence telle que montrée dans l'IS N° 1 ou 2 d'autre part, étant de manière dépendante 2, 3 ou 4.

7. Utilisation d'une composition selon la revendication 5, qui comprend un virus dans le génome duquel est inséré un fragment d'ADN codant pour un polypeptide reconnu par l'anticorps H23, ayant pour séquence (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n étant un nombre de 1 à 80.

8. Utilisation d'une composition selon la revendication 7, qui comprend un virus dans le génome duquel est inséré un fragment d'ADN codant pour un polypeptide reconnu par l'anticorps H23, ayant pour séquence (i) la séquence telle que montrée dans l'IS N° 1 commençant avec le résidu thréonine en position 1 et finissant avec le résidu leucine en position 414 + (20 x n) ou (ii) la séquence telle que montrée dans l'IS N° 2 commençant avec le résidu thréonine en position 1 et finissant avec le résidu proline en position 246 + (20 x n) ; le nombre n étant 2, 3 ou 4.

9. Utilisation d'une composition selon l'une quelconque des revendications 5 à 8, dans laquelle le virus dans le génome duquel est inséré un fragment d'ADN codant pour un polypeptide reconnu par l'anticorps H23 est un poxvirus.

10. Utilisation d'une composition selon la revendication 9, dans laquelle le poxvirus dans le génome duquel est inséré un fragment d'ADN codant pour un polypeptide reconnu par l'anticorps H23 est le virus de la vaccine.

11. Utilisation d'une composition selon l'une quelconque des revendications 5 à 8, dans laquelle le virus dans le génome duquel est inséré un fragment d'ADN codant pour un polypeptide reconnu par l'anticoprs H23 est un adénovirus.

## Patentansprüche (Patentansprüche für folgende(n) Vertragsstaat(en): AT, BE, CH, DE, DK, FR, GB, IT, LU, LI, NL, SE)

1. Pharmazeutische Zusammensetzung zur Behandlung oder Prävention eines malignen Tumors, die als therapeutisches Mittel ein durch den Antikörper H23 erkanntes Polypeptid in Verbindung mit einem unter pharmazeutischem Gesichtspunkt verträglichen Verdünner oder Träger umfaßt, wobei das durch den Antikörper H23 erkannte Polypeptid eine n-mal wiederholte Sequenz der Formel (1) umfaßt: Pro-Gly-Ser-Thr-Ala-Pro-X₁-Ala-His-Gly-Val-Thr-Ser-Ala-Pro-Asp-Y-Arg-Pro-X₂, wobei X₁ und X₂ unabhängig voneinander Pro oder Ala sind und Y Thr oder Asn ist, mit Ausnahme der Sequenz, in der X₁, X₂ und Y jeweils Pro, Ala und Thr darstellen, wobei das Polypeptid eine vollständige Sequenz aufweist, die ein Grad an Homologie von mindestens 80 % mit (i) der in IS Nr. 1 dargestellten Sequenz aufweist, die mit dem Threonin-Rest in Position 1 beginnt und mit dem Leucin-Rest in Position 414 + (20 x n) endet oder (ii) der in IS Nr. 2 dargestellten Sequenz, die mit dem Threonin-Rest in Position 1 beginnt und mit dem Prolin-Rest in Position 246 + (20 x n) endet; wobei die Zahl n unter Bezug auf die Sequenz der Formel (I) des Polypeptids einerseits und unter Bezug auf die in IS Nr. 1 oder 2 dargestellte Sequenz andererseits in abhängiger Weise eine Zahl von 1 bis 80 bedeutet.

2. Zusammensetzung nach Anspruch 1, bei der das vom Antikörper H23 erkannte Polypeptid ein Polypeptid ist, das die n-fach wiederholte Sequenz der Formel (I) umfaßt, deren vollständige Sequenz einen Grad an Homologie von mindestens 80 % mit (i) der in IS Nr. 1 gezeigten Sequenz aufweist, die mit dem Threonin-Rest in Position 1 beginnt und mit dem Leucin-Rest in Position 414 + (20 x n) endet oder (ii) der in IS Nr. 2 dargestellten Sequenz, die mit dem Threonin-Rest in Position 1 beginnt und mit dem Prolin-Rest in Position 246 + (20 x n) endet; wobei die Zahl n unter Bezug auf die Sequenz der Formel (I) des Polypeptids einerseits und unter Bezug auf die in IS Nr. 1 oder 2 dargestellte Sequenz andererseits in abhängiger Weise 2, 3 oder 4 bedeutet.

3. Zusammensetzung nach Anspruch 1, bei der vom Antikörper H23 erkannte Polypeptid als Sequenz (i) die Sequenz aufweist wie in IS Nr. 1 dargestellt, die mit dem Threonin-Rest in Position 1 beginnt und mit dem Leucin-Rest in Position 414 + (20 x n) endet oder (ii) die Sequenz wie in IS Nr. 2 dargestellt, die mit dem Threonin-Rest in Position 1 beginnt und mit dem Prolin-Rest in Position 246 + (20 x n) endet; wobei n eine Zahl von 1 bis 80 ist.

4. Zusammensetzung nach Anspruch 3, bei der das vom Antikörper H23 erkannte Polypeptid als Sequenz (i) die Sequenz aufweist wie in IS Nr. 1 dargestellt, die mit dem Threonin-Rest in Position 1 beginnt und mit dem Leucin-Rest in Position 414 + (20 x n) endet oder (ii) die Sequenz wie in IS Nr. 2 dargestellt, die mit dem Threonin-Rest in Position 1 beginnt und mit dem Prolin-Rest in Position 246 + (20 x n) endet; wobei n 2, 3 oder 4 ist.

5. Pharmazeutische Zusammensetzung zur Behandlung oder Prävention eines malignen Tumors, welche als therapeutischen Wirkstoff ein Virus umfaßt, in dessen Genom ein DNA-Fragment insertiert ist, das für ein vom Antikörper H23 erkanntes Polypeptid kodiert, wobei das Polypeptid eine n-mal wiederholte Sequenz der Formel (I) umfaßt: Pro-Gly-Ser-Thr-Ala-Pro-X₁-Ala-His-Gly-Val-Thr-Ser-Ala-Pro-Asp-Y-Arg-Pro-X₂, wobei X₁ und X₂ unabhängig voneinander Pro oder Ala sind und Y Thr oder Asn ist, und dessen vollständige Sequenz einen Grad an Homologie von mindestens 80 % mit (i) der in IS Nr. 1 dargestellten Sequenz aufweist, die mit dem Threonin-Rest in Position 1 beginnt und mit dem Leucin-Rest in Position 414 + (20 x n) endet oder (ii) der in IS Nr. 2 dargestellten Sequenz, die mit dem Threonin-Rest in Position 1 beginnt und mit dem Prolin-Rest in Position 246 + (20 x n) endet; wobei die Zahl n unter Bezug auf die Sequenz der Formel (I) des Polypeptids einerseits und unter Bezug auf die in IS Nr. 1 oder 2 dargestellte Sequenz andererseits in abhängiger Weise eine Zahl von 1 bis 80 ist, wobei das DNA-Fragment unter der Kontrolle von geeigneten Transkriptions- und Translations-Signalen steht.

6. Zusammensetzung nach Anspruch 5, die ein Virus umfaßt, in dessen Genom ein DNA-Fragment insertiert ist, welches für ein vom Antikörper H23 erkanntes Polypeptid kodiert, umfassend die n-fach wiederholte Sequenz der Formel (I) umfaßt, dessen vollständige Sequenz einen Grad an Homologie von mindestens 80 % mit (i) der in IS Nr. 1 gezeigten Sequenz aufweist, die mit dem Threonin-Rest in Position 1 beginnt und mit dem Leucin-Rest in Position 414 + (20 x n) endet oder (ii) der in IS Nr. 2 dargestellten Sequenz, die mit dem Threonin-Rest in Position 1 beginnt und mit dem Prolin-Rest in Position 246 + (20 x n) endet; wobei die Zahl n unter Bezug auf die Sequenz der Formel (I) des Polypeptids einerseits und unter Bezug auf die in IS Nr. 1 oder 2 dargestellte Sequenz andererseits in abhängiger Weise 2, 3 oder 4 bedeutet.

7. Zusammensetzung nach Anspruch 5, die ein Virus umfaßt, in dessen Genom ein DNA-Fragment insertiert ist, welches für ein vom Antikörper H23 erkanntes Polypeptid kodiert und als Sequenz (i) die in IS Nr. 1 gezeigte Sequenz aufweist, die mit dem Threonin-Rest in Position 1 beginnt und mit dem Leucin-Rest in Position 414 + (20 x n) endet oder (ii) die in IS Nr. 2 dargestellte Sequenz, die mit dem Threonin-Rest in Position 1 beginnt und mit dem Prolin-Rest in Position 246 + (20 x n) endet; wobei die Zahl n eine Zahl von 1 bis 80 bedeutet.

8. Zusammensetzung nach Anspruch 7, die ein Virus umfaßt, in dessen Genom ein DNA-Fragment insertiert ist, welches für ein vom Antikörper H23 erkanntes Polypeptid kodiert und als Sequenz (i) die in IS Nr. 1 gezeigte Sequenz aufweist, die mit dem Threonin-Rest in Position 1 beginnt und mit dem Leucin-Rest in Position 414 + (20 x n) endet oder (ii) die in IS Nr. 2 dargestellte Sequenz, die mit dem Threonin-Rest in Position 1 beginnt und mit dem Prolin-Rest in Position 246 + (20 x n) endet; wobei die Zahl n 2, 3 oder 4 bedeutet.

9. Zusammensetzung nach einem der Ansprüche 5 bis 8, bei der das Virus, in dessen Genom ein DNA-Fragment insertiert ist, welches für ein vom Antikörper H23 erkanntes Polypeptid kodiert, ein Pockenvirus ist.

10. Zusammensetzung nach Anspruch 9, wobei das Pockenvirus, in dessen Genom ein DNA-Fragment insertiert ist, welches für ein vom Antikörper H23 erkanntes Polypeptid kodiert, das Virus des Impfstoffs ist.

11. Zusammensetzung nach einem der Ansprüche 5 bis 8, in der das Virus, in dessen Genom ein DNA-Fragment insertiert ist, welches für ein vom Antikörper H23 erkanntes Polypeptid kodiert, ein Adenovirus ist.

## Patentansprüche (Patentansprüche für folgende(n) Vertragsstaat(en): ES, GR)

1. Verwendung einer pharmazeutischen Zusammensetzung zur Herstellung eines Medikamentes zur Behandlung oder Prävention eines malignen Tumors, wobei die Zusammensetzung als therapeutisches Mittel ein vom Antikörper H23 erkanntes Polypeptid in Verbindung mit einem unter pharmazeutischem Gesichtspunkt verträglichen Verdünner oder Träger umfaßt, wobei das vom Antikörper H23 erkannte Polypeptid eine n-mal wiederholte Sequenz der Formel (I) umfaßt: Pro-Gly-Ser-Thr-Ala-Pro-X₁-Ala-His-Gly-Val-Thr-Ser-Ala-Pro-Asp-Y-Arg-Pro-X₂, wobei X₁ und X₂ unabhängig voneinander, Pro oder Ala sind und Y Thr oder Asn ist, mit Ausnahme der Sequenz, in der X₁, X₂ und Y jeweils Pro, Ala und Thr darstellen, wobei das Polypeptid eine vollständige Sequenz aufweist, die einen Grad an Homologie von mindestens 80 % mit (i) der in IS Nr. 1 gezeigten Sequenz aufweist, die mit dem Threonin-Rest in Position 1 beginnt und mit dem Leucin-Rest in Position 414 + (20 x n) endet oder (ii) der in IS Nr. 2 dargestellten Sequenz, die mit dem Threonin-Rest in Position 1 beginnt und mit dem Prolin-Rest in Position 246 + (20 x n) endet; wobei die Zahl n unter Bezug auf die Sequenz der Formel (I) des Polypeptids einerseits und unter Bezug auf die in IS Nr. 1 oder 2 dargestellte Sequenz andererseits in abhängiger Weise eine Zahl von 1 bis 80 bedeutet.

2. Verwendung einer pharmazeutischen Zusammensetzung nach Anspruch 1, bei der das vom Antikörper H23 erkannte Polypeptid ein Polypeptid-ist, das die n-fach wiederholte Sequenz der Formel (I) umfaßt, deren vollständige Sequenz einen Grad an Homologie von mindestens 80 % mit (i) der in IS Nr. 1 gezeigten Sequenz aufweist, die mit dem Threonin-Rest in Position 1 beginnt und mit dem Leucin-Rest in Position 414 + (20 x n) endet oder (ii) der in IS Nr. 2 dargestellten Sequenz, die mit dem Threonin-Rest in Position 1 beginnt und mit dem Prolin-Rest in Position 246 + (20 x n) endet; wobei die Zahl n unter Bezug auf die Sequenz der Formel (I) des Polypeptids einerseits und unter Bezug auf die in IS Nr. 1 oder 2 dargestellte Sequenz andererseits in abhängiger Weise 2, 3 oder 4 bedeutet.

3. Verwendung einer Zusammensetzung nach Anspruch 1, bei der das vom Antikörper H23 erkannte Polypeptid als Sequenz (i) die Sequenz aufweist wie in IS Nr. 1 dargestellt, die mit dem Threonin-Rest in Position 1 beginnt und mit dem Leucin-Rest in Position 414 + (20 x n) endet oder (ii) die Sequenz wie in IS Nr. 2 dargestellt, die mit dem Threonin-Rest in Position 1 beginnt und mit dem Prolin-Rest in Position 246 + (20 x n) endet; wobei n eine Zahl von 1 bis 80 ist.

4. Verwendung einer Zusammensetzung nach Anspruch 3, bei der das vom Antikörper H23 erkannte Polypeptid als Sequenz (i) die Sequenz aufweist wie in IS Nr. 1 dargestellt, die mit dem Threonin-Rest in Position 1 beginnt und mit dem Leucin-Rest in Position 441 + (20 x n) endet oder (ii) die Sequenz wie in IS Nr. 2 dargestellt, die mit dem Threonin-Rest in Position 1 beginnt und mit dem Prolin-Rest in Position 246 + (20 x n) endet; wobei n 2, 3 oder 4 ist.

5. Verwendung einer pharmazeutischen Zusammensetzung zur Behandlung oder Prävention eines malignen Tumors, wobei die Zusammensetzung als therapeutischen Wirkstoff ein Virus umfaßt, in dessen Genom ein DNA-Fragment insertiert ist, das für ein vom Antikörper H23 erkanntes Polypeptid kodiert, wobei das Polypeptid eine n-mal wiederholte Sequenz der Formel (I) umfaßt: Pro-Gly-Ser-Thr-Ala-Pro-X₁-Ala-His-Gly-Val-Thr-Ser-Ala-Pro-Asp-Y-Arg-Pro-X₂, wobei X₁ und X₂ unabhängig voneinander Pro oder Ala sind und Y Thr oder Asn ist, und dessen vollständige Sequenz einen Grad der Homologie von mindestens 80 % mit (i) der in IS Nr. 1 dargestellten Sequenz aufweist, die mit dem Threonin-Rest in Position 1 beginnt und mit dem Leucin-Rest in Position 414 + (20 x n) endet oder (ii) der in IS Nr. 2 dargestellten Sequenz, die mit dem Threonin-Rest in Position 1 beginnt und mit dem Prolin-Rest in Position 246 + (20 x n) endet; wobei die Zahl n unter Bezug auf die Sequenz der Formel (I) des Polypeptids einerseits und unter Bezug auf die in IS Nr. 1 oder 2 dargestellte Sequenz andererseits in abhängiger Weise eine Zahl von 1 bis 80 ist, wobei das DNA-Fragment unter der Kontrolle von geeigneten Transkriptions- und Translations-Signalen steht.

6. Verwendung einer Zusammensetzung nach Anspruch 5, die ein Virus umfaßt, in dessen Genom ein DNA-Fragment insertiert ist, welches für ein vom Antikörper H23 erkanntes Polypeptid kodiert, umfassend die n-fach wiederholte Sequenz der Formel (I), dessen vollständige Sequenz einen Grad an Homologie von mindestens 80 % mit (i) der in IS Nr. 1 gezeigten Sequenz aufweist, die mit dem Threonin-Rest in Position 1 beginnt und mit dem Leucin-Rest in Position 414 + (20 x n) endet oder (ii) der in IS Nr. 2 dargestellten Sequenz, die mit dem Threonin-Rest in Position 1 beginnt und mit dem Prolin-Rest in Position 246 + (20 x n) endet; wobei die Zahl n unter Bezug auf die Sequenz der Formel (I) des Polypeptids einerseits und unter Bezug auf die in IS Nr. 1 oder 2 dargestellte Sequenz andererseits in abhängiger Weise 2, 3 oder 4 bedeutet.

7. Verwendung einer Zusammensetzung nach Anspruch 5, die ein Virus umfaßt, in dessen Genom ein DNA-Fragment insertiert ist, welches für ein vom Antikörper H23 erkanntes Polypeptid kodiert und als Sequenz (i) die in IS Nr. 1 gezeigte Sequenz aufweist, die mit dem Threonin-Rest in Position 1 beginnt und mit dem Leucin-Rest in Position 414 + (20 x n) endet oder (ii) die in IS Nr. 2 dargestellte Sequenz, die mit dem Threonin-Rest in Position 1 beginnt und mit dem Prolin-Rest in Position 246 + (20 x n) endet; wobei die Zahl n eine Zahl von 1 bis 80 bedeutet.

8. Verwendung einer Zusammensetzung nach Anspruch 7, die ein Virus umfaßt, in dessen Genom ein DNA-Fragment insertiert ist, welches für ein vom Antikörper H23 erkanntes Polypeptid kodiert und als Sequenz (i) die in IS Nr. 1 gezeigte Sequenz aufweist, die mit dem Threonin-Rest in Position 1 beginnt und mit dem Leucin-Rest in Position 414 + (20 x n) endet oder (ii) die in IS Nr. 2 dargestellte Sequenz, die mit dem Threonin-Rest in Position 1 beginnt und mit dem Prolin-Rest in Position 246 + (20 x n) endet; wobei die Zahl n 2, 3 oder 4 bedeutet.

9. Verwendung einer Zusammensetzung nach einem der Ansprüche 5 bis 8, bei der der Virus, in dessen Genom ein DNA-Fragment insertiert ist, welches für ein vom Antikörper H23 erkanntes Polypeptid kodiert, ein Pockenvirus ist.

10. Verwendung einer Zusammensetzung nach Anspruch 9, wobei das Pockenvirus, in dessen Genom ein DNA-Fragment insertiert ist, welches für ein vom Antikörper H23 erkanntes Polypeptid kodiert, das Virus des Impfstoffs ist.

11. Verwendung einer Zusammensetzung nach einem der Ansprüche 5 bis 8, in der das Virus, in dessen Genom ein DNA-Fragment insertiert ist, welches für ein vom Antikörper H23 erkanntes Polypeptid kodiert, ein Adenovirus ist.

## Claims (Claims for the following Contracting State(s): AT, BE, CH, DE, DK, FR, GB, IT, LU, LI, NL, SE)

1. A pharmaceutical composition for treating or preventing a malignant tumour, which comprises, as therapeutic agent, a polypeptide recognized by antibody H23, in combination with a diluent or vehicle which is acceptable from a pharmaceutical standpoint and in which the polypeptide recognized by antibody H23 comprises a sequence repeated n times and of formula (I): Pro-Gly-Ser-Thr-Ala-Pro-X₁-Ala-His-Gly-Val-Thr-Ser-Ala-Pro-Asp-Y-Arg-PRo-X₂ in which X₁ and X₂ are, independently, Pro or Ala and Y is Thr or Asn, excluding the sequence in which X₁, X₂ and Y represent Pro, Ala and Thr respectively, the said polypeptide having a complete sequence which exhibits a degree of homology of at least 80% with (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n relating to the sequence of formula (I) of the said polypeptide on the one hand and that relating to the sequence as shown in SI No. 1 or 2 on the other hand being, in a dependent manner, a number from 1 to 80.

2. A composition according to Claim 1, in which the polypeptide recognized by antibody H23 is a polypeptide comprising the sequence of formula (I) repeated n times whose complete sequence exhibits a degree of homology of at least 80% with (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n relating to the sequence of formula (I) of the said polypeptide on the one hand and that relating to the sequence as shown in SI No. 1 or 2 on the other hand being, in a dependent manner, 2, 3 or 4.

3. A composition according to Claim 1, in which the polypeptide recognized by antibody H23 has as its sequence (i) the sequence as shown in SI No. 1 beginning. with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); n being a number from 1 to 80.

4. A composition according to Claim 3, in which the polypeptide recognized by antibody H23 has as its sequence (i) the-sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); n being 2,3 or 4.

5. A pharmaceutical composition for treating or preventing a malignant tumour, which comprises, as therapeutic agent, a virus into the genome of which a DNA fragment coding for a polypeptide recognized by antibody H23 is inserted, the said polypeptide comprising a sequence repeated n times of formula (I): Pro-Gly-Ser-Thr-Ala-Pro-X₁-Ala-His-Gly-Val-Thr-Ser-Ala-Pro-Asp-Y-Arg-Pro-X₂ in which X₁ and X₂ are, independently, Pro or Ala and Y is Thr or Asn and whose complete sequence exhibits a degree of homology of at least 80% with (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n relating to the sequence of formula (I) of the said polypeptide on the one hand and that relating to the sequence as shown in SI No. 1 or 2 on the other hand being, in a dependent manner, a number from 1 to 80, the said DNA fragment being placed under the control of suitable transcription and translation signals.

6. A composition according to Claim 5, which comprises a virus into the genome of which a DNA fragment coding for a polypeptide recognized by antibody H23 is inserted, comprising the sequence of formula (I) repeated n times and whose complete sequence exhibits a degree of homology of at least 80% with (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n relating to the sequence of formula (I) of the said polypeptide on the one hand and that relating to the sequence as shown in SI No. 1 or 2 on the other hand being, in a dependent manner, 2, 3 or 4.

7. A composition according to Claim 5, which comprises a virus into the genome of which a DNA fragment coding for a polypeptide recognized by antibody H23 is inserted, having as its sequence (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n being a number from 1 to 80.

8. A composition according to Claim 7, which comprises a virus into the genome of which a DNA fragment coding for a polypeptide recognized by antibody H23 is inserted, having as its sequence (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n being 2, 3 or 4.

9. A composition according to any one of Claims 5 to 8, in which the virus into the genome of which a DNA fragment coding for a polypeptide recognized by antibody H23 is inserted is a poxvirus.

10. A composition according to Claim 9, in which the poxvirus into the genome of which a DNA fragment coding for a polypeptide recognized by antibody H23 is inserted is vaccinia virus.

11. A composition according to any one of Claims 5 to 8, in which the virus into the genome of which a DNA fragment coding for a polypeptide recognized by antibody H23 is inserted is an adenovirus.

## Claims (Claims for the following Contracting State(s): ES, GR)

1. Use of a pharmaceutical composition for the preparation of a medicament intended for the treament or prevention of a malignant tumour, the said composition comprising, as therapeutic agent, a polypeptide recognized by antibody H23, in combination with a diluent or vehicle which is acceptable from a pharmaceutical standpoint and in which the polypeptide recognized by antibody H23 comprises a sequence repeated n times and of formula (I): Pro-Gly-Ser-Thr-Ala-Pro-X₁-Ala-His-Gly-Val-Thr-Ser-Ala-Pro-Asp-Y-Arg-Pro-X₂ in which X₁ and X₂ are, independently, Pro or Ala and Y is Thr or Asn, excluding the sequence in which X₁, X₂ and Y represent Pro, Ala and Thr respectively, the said polypeptide having a complete sequence which exhibits a degree of homology of at least 80% with (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n relating to the sequence of formula (I) of the said polypeptide on the one hand and that relating to the sequence as shown in SI No. 1 or 2 on the other hand being, in a dependent manner, a number from 1 to 80.

2. Use of a pharmaceutical composition according to Claim 1, in which the polypeptide recognize by antibody H23 is a polypeptide comprising the sequence of formula (I) repeated n times whose complete sequence exhibits a degree of homology of at least 80% with (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n relating to the sequence of formula (I) of the said polypeptide on the one hand and that relating to the sequence as shown in SI No. 1 or 2 on the other hand being, in a dependent manner, 2, 3 or 4.

3. Use of a composition according to Claim 1, in which the polypeptide recognized by antibody H23 has as its sequence (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); n being a number from 1 to 80.

4. Use of a composition according to Claim 3, in which the polypeptide recognized by antibody H23 has as its sequence (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 245 + (20 × n); n being 2,3 or 4.

5. Use of a pharmaceutical composition for the preparation of a medicament intended for the treatment or the prevention of a malignant tumour, the said composition comprising, as therapeutic agent, a virus into the genome of which a DNA fragment coding for a polypeptide recognized by antibody H23 is inserted, the said polypeptide comprising a sequence repeated n times of formula (I): Pro-Gly-Ser-Thr-Ala-Pro-X₁-Ala-His-Gly-Val-Thr-Ser-Ala-Pro-Asp-Y-Arg-Pro-X₂ in which X₁ and X₂ are, independently, Pro or Ala and Y is Thr or Asn and whose complete sequence exhibits a degree of homology of at least 80% with (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n relating to the sequence of formula (I) of the said polypeptide on the one hand and that relating to the sequence as shown in SI No. 1 or 2 on the other hand being, in a dependent manner, a number from 1 to 80, the said DNA fragment being placed under the control of suitable transcription and translation signals.

6. Use of a composition according to Claim 5, which comprises a virus into the genome of which a DNA fragment coding for a polypeptide recognized by antibody H23 is inserted, comprising the sequence of formula (I) repeated n times and whose complete sequence exhibits a degree of homology of at least 80% with (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n relating to the sequence of formula (I) of the said polypeptide on the one hand and that relating to the sequence as shown in SI No. 1 or 2 on the other hand being, in a dependent manner, 2, 3 or

7. Use of a composition according to Claim 5, which comprises a virus into the genome of which a DNA fragment coding for a polypeptide recognized by antibody H23 is inserted, having as its sequence (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n being a number from 1 to 80.

8. Use of a composition according to Claim 7, which comprises a virus into the genome of which a DNA fragment coding for a polypeptide recognized by antibody H23 is inserted, having as its sequence (i) the sequence as shown in SI No. 1 beginning with the threonine residue at position 1 and ending with the leucine residue at position 414 + (20 × n) or (ii) the sequence as shown in SI No. 2 beginning with the threonine residue at position 1 and ending with the proline residue at position 246 + (20 × n); the number n being 2, 3 or 4.

9. Use of a composition according to any one of Claims 5 to 8, in which the virus into the genome of which a DNA fragment coding for a polypeptide recognized by antibody H23 is inserted is a poxvirus.

10. Use of a composition according to Claim 9, in which the poxvirus into the genome of which a DNA fragment coding for a polypeptide recognized by antibody H23 is inserted is vaccinia virus.

11. Use of a composition according to any one of Claims 5 to 8, in which the virus into the genome of which a DNA fragment coding for a polypeptide recognized by antibody H23 is inserted is an adenovirus.
